# EUROPÄISCHE PATENTSCHRIFT

(11) **EP 2 255 170 B1**
(45) Veröffentlichungstag und Bekanntmachung des Hinweises auf die Patenterteilung: **22.05.2013**
(21) Anmeldenummer: 09721788.9
(22) Anmeldetag: 18.03.2009
(51) Int. Cl.: G01L 5/16, G01L 1/24

(54) **OPTISCHES MESSELEMENT MIT EINSTÜCKIGER STRUKTUR**
OPTICAL MEASURING ELEMENT HAVING A SINGLE-PIECE STRUCTURE
ÉLÉMENT DE MESURE OPTIQUE À STRUCTURE MONOBLOC

(30) Priorität: 19.03.2008 CH 418082008; 09.04.2008 US 43439
(43) Veröffentlichungstag der Anmeldung: 01.12.2010
(73) Patentinhaber: Kistler Holding AG, 8408 Winterthur (CH); Sensoptic SA, 6616 Losone (CH)
(72) Erfinder: BERTHOLDS, Axel, CH-6653 Verscio (CH); LLOSAS, Pere, CH-6648 Minusio (CH); HENEIN, Simon, CH-2000 Neuchâtel (CH)
(86) Internationale Anmeldenummer: PCT/CH2009/000096
(87) Internationale Veröffentlichungsnummer: WO 2009/114955

(56) Entgegenhaltungen:
- WO-A-2007/015139
- US-A1- 2008 009 750
- PEIRS J ET AL: "A micro optical force sensor for force feedback during minimally invasive robotic surgery" SENSORS AND ACTUATORS A, ELSEVIER SEQUOIA S.A., LAUSANNE, CH, Bd. 115, Nr. 2-3, 21. September 2004 (2004-09-21), Seiten 447-455, XP004562104 ISSN: 0924-4247 in der Anmeldung erwähnt

## Beschreibung

### Technisches Gebiet

Die Erfindung betrifft ein optisches Messelement zum Messen von Kräften in mindestens einer Richtung, wobei das Messelement eine einstückige Struktur mit einer Aussenwand umfasst und mit darin eingebrachten Einschnitten. Die Einschnitte definieren eine oder mehrere elastisch flexible Zonen in der Struktur, welche die einzige Verbindung zwischen einem ersten und einem zweiten Bereich der Struktur darstellen. Für optische Abstandsmessungen zwischen den beiden Bereichen der Struktur sind ein oder mehrere Lichtleiter mit jeweils einem Ende derart an einem Bereich der Struktur angebracht, dass sich nahe gegenüber seinen Enden reflektierende Flächen befinden, die fest mit einem anderen Bereich verbunden sind.

### Stand der Technik

Optische Kraftmesselemente haben gegenüber anderen Kraftmesselementen, wie solchen auf DMS beruhend, den Vorteil, dass sie keine elektrischen Leitungen und Verbindungen enthalten. Dies ist insbesondere vorteilhaft beim Einsatz in Gebieten, in denen elektromagnetische Störungen zu erwarten sind. Zudem lassen sich solche Messelemente wegen den elektrischen Kontaktierungen nur begrenzt miniaturisieren. Für medizinische Zwecke, speziell für invasive Anwendungen, sind optische Kraftmesselemente geeignet. Andere Anwendungsbereiche sind beispielsweise die Robotertechnologie. Die Unterschiede der optisch gemessenen Distanzen sind proportional zu den einwirkenden Kräften, welche die Struktur verformen.

In der WO 2007/015139 ist ein Katheter mit einer Sensorspitze beschrieben, an deren Ende ein optisches Kraftmesselement angebracht ist. Diese Sensorspitze kann in einem menschlichen Organ, beispielsweise in einem Herzen, an dessen Wand gebracht werden und ermittelt dort die durch die Organwand aufgebrachte dreidimensionale Kraft auf die Sensorspitze. So kann ein sogenanntes "Mapping" von Gefässen und Organen durchgeführt werden.

In der Veröffentlichung von J. Peirs, J. Clijnen, P. Herijgers, D. Reynaerts, H. Van Brussel, B. Corteville und S. Boone "Design of an Optical Force Sensor for Force Feedback during Minimally. Invasive Robotic Surgery" wird ein optischer Sensor beschrieben, der beispielsweise für die oben beschriebene Anwendung eingesetzt werden kann. Er beschreibt eine elastische äussere zylindrische Struktur, in der drei Lichtleiter mit drei Faserenden mittig eingelagert sind. Diese messen die Distanz zu einem festen Ende, das je nach Krafteinwirkung auf den oberen Teil der Struktur näher bei den Faserenden ist. Nachteilig an diesem Sensor ist die begrenzte Möglichkeit zur Miniaturisierung. Zudem ist die Messung der Kräfte in die drei Richtungen x, y und z stets gekoppelt.

In der US 2008/0009750 A1 ist eine Katheterspitze mit eingebauter, eingangs erwähnte Struktur angegeben. Sie ist identisch mit der Struktur, die bereits in der Veröffentlichung von Peirs et al beschrieben wurde. Sie besteht aus einem Rohr, das durch radial angebrachte Einschnitte im mittleren Bereich zu einer flexiblen Struktur geschnitten wurde. Diese weist eine Vielzahl von Säulen auf, die abwechslungsweise an einem oberen und an einem unteren Ring angebracht sind, wobei jeweils ein benachbartes Säulenpaar mit zwei flexiblen Stegen miteinander verbunden ist. Im unteren Ring sind Lichtleiter in Öffnungen angeordnet, die den Abstand zu den unteren Enden der oben angebrachten Säulen oder zu den Stegen messen können, um dadurch auf die auf den oberen Ring einwirkenden Kräfte zu schliessen.

Da alle Einschnitte und somit alle Ränder der Säulen und der elastischen Stege radial zur Rohrachse verlaufen, sind die Säulen an der Rohraussenwand dicker als an der Rohrinnwand, und die Stege sind an der Rohraussenwand weicher als an der Rohrinnwand, weil sie dort kürzer sind. Aus diesem Grund muss die Wahddicke des Rohres dünn ausgestaltet sein im Verhältnis zum Aussendurchmesser des Rohres, etwa 1:10, um die Flexibilität der elastischen Stege zu gewährleisten. Die angegebenen Masse von 0.5 mm Wandstärke und 5 mm Rohrdurchmesser können nicht unterschritten werden, da die Struktur sonst zu labil werden würde. Für viele Anwendungen, beispielsweise auch für Katheter, werden aber kleinere Sensoren gefordert.

Ein weiterer Nachteil dieser Struktur ist, dass sowohl eine axial als auch eine radial auf die Struktur wirkende Kraft stets eine Torsion auslöst, bedingt durch den spiralförmigen Aufbau der Struktur. Diese Struktur-bedingte Torsion erschwert die Umrechnung von den ermittelten Abstandsmessungen zu den dreidimensional einwirkenden Kräften. Insbesondere ist im beschriebenen Katheter nicht ersichtlich, wie eine auf die Katheterspitze einwirkende Kraft auf das Rohr übertragen wird, da ausser über die Aussenwand keine Verbindung zur Katheterspitze vorgesehen ist. Eine axiale Last würde sich demnach kaum auf das Messelement übertragen.

### Darstellung der Erfindung

Aufgabe der vorliegenden Erfindung ist es, ein Messelement gemäss dem eingangs beschriebenen Sensor anzugeben, welches einfacher und kleiner hergestellt werden kann und zuverlässige Rückschlüsse von den gemessenen Daten zu den vorherrschenden Kräften liefert. Zudem soll die Möglichkeit bestehen, die Kräfte in x, y und z ungekoppelt zu messen.

Die Aufgabe wird gelöst durch die Kennzeichen des unabhängigen Patentanspruchs.

Die der Erfindung zugrunde liegende Idee besteht darin, dass beim erfindungsgemässen Messelement die Lichtleiter an der Aussenwand, das heisst an der äusseren Oberfläche der Struktur, angeordnet sind. Dadurch sind sie einerseits einfacher anzubringen, da sie von aussen angebracht und/oder fixiert werden können. Vor allem aber verlangt die Struktur somit nicht unbedingt nach einer zentralen Bohrung, wodurch die Struktur robuster, kompakter und kleiner ausgestaltet werden kann. Zudem besteht die der Erfindung zugrunde liegende Idee darin, dass jeder Einschnitt aus parallelen Rändern besteht. Durch diese flächigen Einschnitte muss die Struktur nicht zwingend rohrförmig sein sondern kann solide ausgestaltet sein, wobei eine zentrale Bohrung durchaus vorgesehen sein kann. Diese sollte für miniaturisierte Abwendungen maximal etwa die Hälfte des Durchmessers der Gesamtstruktur ausmachen, damit das verbleibende Material noch die geforderte Festigkeit aufweist. Der Durchmesser einer erfindungsgemässen Struktur misst weniger als die Hälfte eines Durchmessers einer Struktur nach dem Stand der Technik.

Zusätzlich wird die Struktur als Messelement in einer bevorzugten Ausführungsform noch genauer durch die Schaffung von unabhängigen elastisch flexiblen Zonen, welche jeweils nur in eine Richtung x, y oder z deformierbar sind, da die einzelnen Kraftkomponenten entkoppelt gemessen werden können.

### Kurze Beschreibung der Zeichnungen

Im Folgenden wird die Erfindung unter Beizug der Zeichnungen näher erklärt. Es zeigen
- Fig. 1: eine fotografische Darstellung eines erfindungsgemässen Messelements;
- Fig. 2: eine schematische Darstellung einer Seitenansicht des erfindungsgemässen Sensors nach Fig. 1;
- Fig. 3: eine schematische Darstellung eines erfindungsgemässen Sensors in einer alternativen, gekoppelten Ausführung;
- Fig. 4: einen Querschnitt der Darstellung nach Fig. 3;
- Fig. 5: eine schematische Darstellung eines erfindungsgemässen Sensors in einer alternativen, teilweise gekoppelten Ausführung.

### Wege zur Ausführung der Erfindung

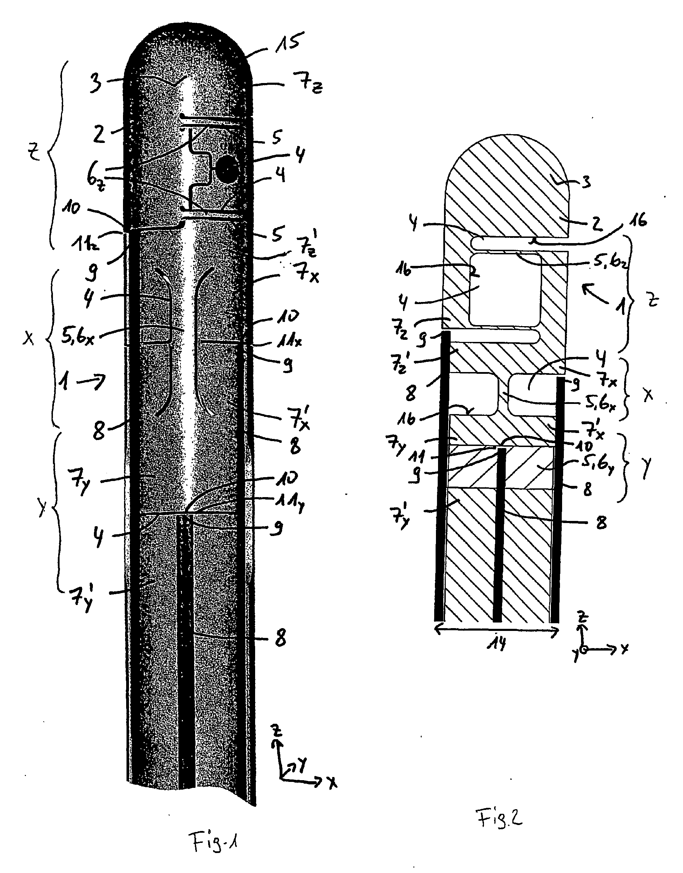

Die Fig. 1 zeigt eine perspektivische fotografische Darstellung eines erfindungsgemässen Messelements. Das dargestellte optische Messelement 1 eignet sich zum Messen von Kräften in drei Richtungen x, y und z, wobei z in Axialrichtung des Messelements verläuft und x und y senkrecht dazu, in radialer Richtung verlaufen.

Die Fig. 2 zeigt dasselbe Messelement wie Fig. 1, jedoch in schematischer, vereinfachter Darstellung. In dieser Darstellung wurde unnötiges Material in der Struktur weggelassen, wodurch die Funktionsweise einfacher verständlich wird. Insbesondere die Verbindungen, welche die Struktur in Bereiche teilt, sind klar ersichtlich. Die Fig. 2 Kann somit als äquivalent der Fig. 1 betrachtet werden, wobei die Grössenverhältnisse in Fig. 1 der Realität entsprechen, die der Fig. 2 aber nicht.

Das Messelement 1 umfasst eine einstückige Struktur 2 mit einer äusseren Oberfläche, der Aussenwand 3, und mit in der Struktur 2 eingebrachten Einschnitten 4. Diese Einschnitte 4 definieren mehrere elastisch flexible Zonen 5 in der Struktur 2, welche die einzigen Verbindungen 6x, 6y und 6z zwischen jeweils einem ersten Bereich 7x und einem zweiten Bereich 7x', resp. zwischen den Bereichen 7y und 7y' und zwischen 7z und 7z' der Struktur 2 darstellen. Die flexiblen Zonen 5 sind vorzugsweise in allen Versionen als dünne Stege ausgestaltet. Das Messelement 1 weist insbesondere ein gerundetes Ende auf, welches geeignet ist, die dreidimensional zu messende Kraft aufzunehmen und in die einzelnen Bereiche 7 weiterzuleiten.

In dieser Ausführungsform ist der Bereich 7z' identisch mit 7x und 7x' ist identisch mit 7y. Somit verfügt dieses Messelement über vier durch Verbindungen 6 getrennte Bereiche 7.

Einfachere Messelemente 1 umfassen entsprechend über Einschnitte 4, die nur eine Verbindung 6 aufweisen, welche aus mehreren elastisch flexiblen Zonen 5 bestehen kann. Diese Einschnitte 4 teilen das Messelement 1 in genau zwei Bereiche 7 und 7'. Entsprechend können die Einschnitte 4 das Messelement 1 alternativ auch durch zwei Verbindungen 6z, 6xy in drei Bereiche 7, 7' = 71 und 71' teilen. In dieser Ausführungsform kann eine Verbindung 6 eine flexible Zone in mehr als eine Richtung zulassen.

Zwischen zwei Bereichen 7, 7' ist jeweils ein Spaltabstand 11 durch Einschnitte 4 definiert, der sich verändert, wenn eine Kraft auf die Sensorspitze einwirkt. Bei einer Krafteinwirkung aus der Z-Richtung verändert sich der Spaltabstand 11z, entsprechendes gilt für Krafteinwirkungen aus der x- und y-Richtung.

Für optische Abstandsmessungen zwischen jeweils zwei Bereichen 7, 7' der Struktur 2 ist am zweiten dieser Bereichen 7' ein Lichtleiter 8 mit einem Ende 9 an der Struktur 2 derart angebracht, dass sich gegenüber diesem Ende 9, am ersten Bereich 7, eine reflektierende Fläche 10 befindet, die fest mit diesem ersten Bereich 7 verbunden ist. Aus diesem Lichtleiter 8 ausgestrahltes Licht wird bei einer Messung an der reflektierenden Fläche 10 reflektiert, wonach er wieder in den Lichtleiter 8 eintritt. Ein am Lichteiter angeschlossenes Auswertegerät kann aufgrund der erhaltenen Informationen auf den Spaltabstand 11 und schliesslich auf die auf das Messelement 1 einwirkende Kraft schliessen.

Erfindungsgemäss sind diese Lichtleiter 8 an der Aussenwand oder Aussenseite 3 des Messelements 1 angebracht. Im Gegensatz zum Stand der Technik, bei dem die Lichtleiter im Innern der rohrförmigen Struktur angebracht sind, lässt sich die vorliegende Anordnung einfacher, kostengünstiger und genauer herstellen. Erfindungsgemäss sind zur Aussenwand 3 hin offene Aussparungen an der Struktur 2 angebracht, in welche die Lichtleiter gebracht werden. Die Lichtleiter 8 können dann entweder von der Aussenwand 3 her oder vom unteren Ende her in die Aussparungen der Struktur 2 eingeführt werden. Die Öffnung zur Aussenwand 3 hin ist vor allem wichtig für die Befestigung der Lichtleiter 8 an der Struktur 2. Dieses Fassen und feste Anbringen der Lichtleiter 8 an der Aussenwand 3 ist dadurch einfacher und verlangt nicht nach einem zusätzlichen zentralen Bauteil, in dem die Lichtleiter erst angebracht werden müssen, bevor dieses Bauteil mit der Struktur zu einem Messelement zusammengesetzt wird. Zudem kann durch diese Konstruktionsart der Durchmesser des Messelements 1 deutlich verringert werden, nämlich für Messungen von bis zu 5N bis oder auf weniger als 2.5 mm, was insbesondere für medizinische Anwendungen von grosser Bedeutung ist. Bei Durchmessern von 1.7 bis 2 mm können Kräfte von 0.01 bis 1 N gemessen werden, ohne Gefahr zu laufen, die Struktur zu beschädigen.

Ein weiterer Vorteil kann erreicht werden, wenn, wie in den dargestellten Anordnungen, alle Einschnitte 4 die gesamte Struktur 2 bis zur gegenüberliegenden Aussenwand 3 durchdringen. Zudem werden sie durch Schnitte ausgeführt, die jeder für sich, parallele Ränder 16 definiert. Dadurch muss das Messelement bei der Herstellung nicht rotiert werden, was erstens billiger kommt, und zweitens genauere Schnitte definiert.

Im Gegensatz dazu durchdringen die Einschnitte 4 aus dem bekannten Stand der Technik die rohrförmige Struktur jeweils nur radial, was eine teurere Herstellung hervorruft.

Die Einschnitte 4 werden in der Regel durch Drahterodierung (Wire-EDM; EDM: electrical discharge machining, Funkenerudieren) hergestellt, wodurch Einschnitte 4 erzielt werden, die bis zu 0.05 mm dünn sind.

Erfindungsgemäss werden in einem Messelement 1 mindestens so viele Lichtleiter 8 eingesetzt, wie Richtungen gemessen werden sollen. Bei einem Messelement 1 mit nur einer zu messenden Komponente genügt daher der Einsatz eines einzigen Lichtleiter 8, für zwei Komponenten sind mindestens zwei Lichteliter 8 und für drei Komponenten mindestens drei Lichtleiter 8 an der Struktur anzubringen. Jeder Lichtleiter misst den Abstand zwischen zwei benachbarten Bereichen 7 der einstückigen Struktur 2. Wenn mehr als die notwendige Anzahl von Lichtleitern 8 im Einsatz sind, können entsprechend die Fehler minimiert werden, unter Nutzung bekannter mathematischer Methoden.

Die Verbindungen 6 in Fig. 1 und 2 zwischen zwei benachbarten Strukturen sind entkoppelt und lassen jeweils nur Deformationen in eine der Richtung x, y oder z zu. Somit ist die Struktur durch drei Verbindungen in vier aneinander gereihte Bereiche 7 geteilt, wobei eine erste Verbindung nur Deformationen in Richtung x, eine zweite Verbindung nur Deformationen in Richtung y und eine dritte Verbindung nur Deformationen in Richtung z zulassen.

Entsprechend sind drei Lichtleiter 8 an der Struktur angeordnet, von denen jeder den Abstand eines Spaltabstandes 11 zwischen zwei verschiedenen Bereichen 7 messen kann. In dieser Anordnung sind die Kräfte in die drei Richtungen voneinander entkoppelt.

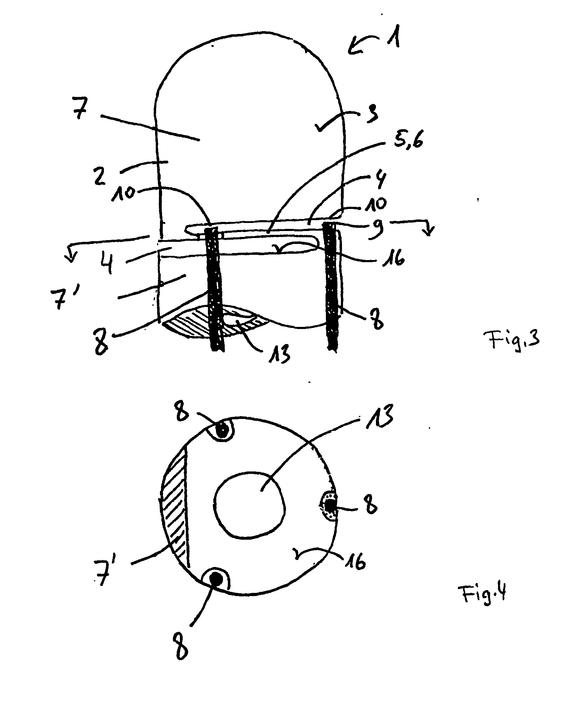

In Fig. 3 ist eine Struktur 2 dargestellt, bei der die Kräfte nicht entkoppelt sind. In Fig. 4 ist ein Querschnitt durch Fig. 3 dargestellt.

In dieser Struktur 2 sind zwei Einschnitte 4 angebracht, welche eine elastisch flexible Zone 5 und somit eine Verbindung 6 definieren. Diese Verbindung 6 lässt Deformationen in alle drei Richtungen x, y und z zu. Andere Einschnitte 4 können entsprechend derart an einer Struktur angebracht sein, dass die dadurch entstandenen Verbindungen 6 Deformationen in zwei der Richtungen x, y und z zulassen.

Die Struktur in Fig. 3 ist durch eine Verbindung in zwei Bereiche 7 geteilt. Drei Lichtleiter 8 messen an verschiednen Stellen den Abstand zwischen diesen beiden Bereichen 7, 7'. Sie sind einigermassen gleichmässig an der Aussenwand 3 angebracht. Zwei von ihnen durchstossen die Verbindung berührungsfrei, an der sie selbst nicht angebracht sind.

In anderen Konfigurationen sind zwischen zwei benachbarten Bereichen 7 jeweils mindestens so viele Lichtleiter 8 angebracht, wie Richtungen gemessen werden sollen.

Die Lichtleiter sind, gleich wie in Fig. 1 und 2 beschrieben, geeignet angebracht, um bei einer Messung den Abstand zur gegenüberliegen, reflektierenden Oberfläche zu messen, welche fest mit dem benachbarten Bereich 7 verbunden ist. Ein Auswertegerät berechnet aus den ermittelten Abstandsmessungen die auf die Struktur 2 aufgetretenen Kräfte in die Richtungen x, y und z. In dieser Anordnung sind die Kräfte gekoppelt. Im Gegensatz zur Anordnung in Fig. 1 ist diese Anordnung vielleicht weniger genau, andererseits ist die Struktur viel kürzer, nämlich etwa 5 mm lang.

Für Verwendungen des Messelements für invasive und minimalinvasive medizinische Zwecke ist eine kurze Länge von grosser Bedeutung.

Solche kurze Messelemente lassen sich durch Katheter mit kleinen Biegeradien führen. Diese sind beispielsweise notwendig für kardiovaskuläre Anwendungen, da der Weg im Herzen zu den Venen über enge Kurven erfolgt.

Wie ebenfalls in Fig. 3 und 4 dargestellt kann die Struktur ein Vollkörper oder ein Hohlkörper sein, der eine zentrale, durchgehende oder nicht durchgehende Aussparung 13 aufweist. Eine solche Aussparung 13 kann auch in einer der anderen erfindungsgemässen Strukturen 2 angebracht sein. Für Katheter Anwendungen sind durchgehende Aussparungen 13 wichtig, um kleine elektrische Kabel und/oder ein feines Rohr für Flüssigkeiten durchzuführen. Zudem kann die Aussparung 13 dazu verwendet werden, Leitungen für Sichtgeräte wie Kabel für eine Kamera oder optische Faserbündel aufzunehmen für endoskopische Anwendungen.

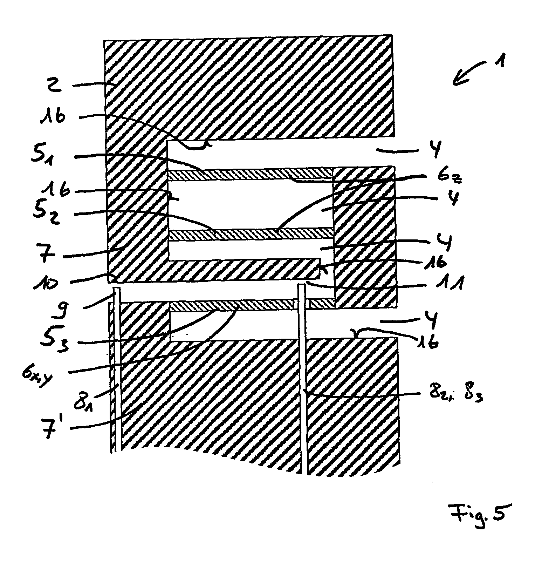

Eine weitere erfindungsgemässe Struktur ist in Fig. 5 schematisch dargestellt. Hier handelt es sich um ein halb entkoppeltes System. Die beiden elastisch flexiblen Zonen 5₁ und 5₂ bilden eine Verbindung 6_{z}, welche eine Bewegung in z-Richtung zulassen, während die elastisch flexible Zone 5₃ eine Verbindung 6_{x,y} für die Richtungen x und y ist. Vollkommene Entkopplung besteht in diesem Fall nicht, entsprechend müssen Korrekturen rechnerisch korrigiert werden. Die Struktur 2 ist natürlich wie alle hier beschriebenen Strukturen einstückig, die verschiedenen Schraffuren in der Struktur dienen lediglich der Verdeutlichung der Funktionsweise. Die Lichtleiter 8₂ und 8₃ sind durch die Verbindung 6 der elastisch flexiblen Zone 5₃ berührungsfrei geführt.

Ein Vorteil der Anordnungen der Fig. 3 und 5 sind, dass die Einschnitte 4 nur in zwei Dimensionen, beispielsweise (x, Z) verlaufen, wodurch sie in einem Arbeitsgang erfolgen können. Die Einschnitte 4 in Fig. 1 müssen in zwei Arbeitsgängen von zwei verschiedenen Richtungen (x, z) und (y, z) her erfolgen. Die Einschnitte nach dem Stand der Technik hingegen müssen radial erfolgen, unter Drehung der Struktur.

Die optischen Abstandsmessungen können insbesondere mit einem photometrischen Messprinzip oder mit Intensitätsmessungen durchgeführt werden. Insbesondere können dazu in allen Anordnungen jeder Lichtleiter 8 aus einem Lichtleiterpaar bestehen. Es hat sich gezeigt, dass interferometrische Messungen, insbesondere mit Weisslicht Interferometrie, viel genauer sind als Intensitätsmessungen.

Zudem kann die Struktur mindestens im Bereich der Einschnitte 4 mit einer Membranhülle 15 zur Abdichtung umschlossen sein. Dies verhindert ein Eindringen von Flüssigkeiten oder anderen Verunreinigungen und ist insbesondere notwendig, wenn die Struktur beispielsweise medizinisch eingesetzt wird. Diese Membranhülle kann vorzugsweise aus Gummi, Teflon oder Silikon bestehen. Falls eine zentrale Aussparung 13 an der Struktur angebracht ist, wird deren innerer Rand ebenfalls abgedichtet, beispielsweise mit einem Teflonrohr.

Ein Überlastschutz verhindert, dass die Enden der Lichtleiter 9 bei Überlast die gegenüberliegenden reflektierenden Flächen berühren und/oder dass die Struktur irreversibel deformiert wird. Ein solcher konstruktionsbedingter Überlastschutz kann aus einem Anschlag zwischen benachbarten Bereichen 7 bestehen, der bei starker Last verhindert, dass ein Ende eines Lichtleiters 9 die Struktur 2 berührt. Alternativ dazu kann der Lichtleiter 8 auch leicht zurückversetzt sein, um eine Kollision zu vermeiden. Vorteilhafterweise ist der Lichtleiter 8 nicht genau an der Stelle montiert, wo der Änderung des Spaltabstandes 11 unter Krafteinfluss am grössten ist und wo demnach ein Verschwinden des Spaltabstandes 11 im Überlastfall zu erwarten ist, sondern nebenan, seitlich versetzt, wo stets ein minimaler Abstand bleibt.

Die Struktur kann aus jedem elektrisch leitenden Material sein, das sich gut und präzise verarbeiten lässt, beispielsweise Titan. Ein alternatives Material in dieser Hinsicht ist leitende Keramik oder ein Halbleitermaterial, insbesondere Silikon.

### Bezugszeichenliste

| | |
|---|---|
| 1 | Optisches Messelement |
| 2 | Einstückige Struktur |
| 3 | Aussenwand, äussere Oberfläche |
| 4 | Einschnitt |
| 5 | Elastisch flexible Zonen |
| 6 | Verbindung |
| 7 | Bereich |
| 8 | Lichtleiter |
| 9 | Ende des Lichtleiters |
| 10 | Reflektierende Flächen |
| 11 | Spaltabstand |
| 12 | Richtungen |
| 13 | Zentrale Aussparung |
| 14 | Durchmesser |
| 15 | Membranhülle |
| 16 | Rand eines Einschnitts |

## Patentansprüche

1. Optisches Messelement (1) zum Messen von Kräften in mindestens einer Richtung (z), wobei das Messelement eine einstückige Struktur (2) mit einer Aussenwand (3) umfasst und mit darin eingebrachten Einschnitten (4), wobei die Einschnitte (4) eine oder mehrere elastisch flexible Zonen (5) in der Struktur (2) definieren, welche die einzige Verbindung (6) zwischen einem ersten und einem zweiten Bereich (7) der Struktur (2) darstellen, und wobei für optische Abstandsmessungen zwischen den beiden Bereichen (7) der Struktur (2) ein oder mehrere Lichtleiter (8) mit jeweils einem Ende (9) derart an einem Bereich (7) der Struktur (2) angebracht sind, dass sich nahe gegenüber seinen Enden (9) reflektierende Flächen (10) befinden, die fest mit einem anderen Bereich (7) verbunden sind, **dadurch gekennzeichnet, dass** die Lichtleiter (8) an der Aussenwand (3) angeordnet sind und dass jeder Einschnitt (4) aus parallelen Rändern (16) besteht.

2. Messelement nach Anspruch 1, **dadurch gekennzeichnet, dass** alle Einschnitte (4) die gesamte Struktur (2) bis zur Aussenwand (3) durchdringen.

3. Messelement nach einem der Ansprüche 1 oder 2, **gekennzeichnet durch** mindestens einen zweiten, vorzugsweise auch einen dritten Lichtleiter (8) für optische Abstandsmessungen zwischen zwei getrennten Bereichen (7) der Struktur (2).

4. Messelement nach Anspruch 3, **dadurch gekennzeichnet, dass** die Struktur (2) durch mehrere Verbindungen (6) mit elastisch flexiblen Zonen (5) in mindestens drei, vorzugsweise vier getrennte Bereiche (7) geteilt ist.

5. Messelement nach Anspruch 4, **dadurch gekennzeichnet, dass** zwischen jeweils zwei benachbarten Bereichen (7) mindestens ein Lichtleiter (8) für optische Abstandsmessungen an der Struktur (2) angebracht ist.

6. Messelement nach Anspruch 4, **dadurch gekennzeichnet, dass** jede Verbindung (6) zwischen zwei benachbarten Strukturen (2) nur Deformationen in eine der Richtung (12) x, y oder z zulassen.

7. Messelement nach Anspruch 6, **dadurch gekennzeichnet, dass** die Struktur (2) durch drei Verbindungen (6) in vier aneinander gereihte Bereiche (7) geteilt ist, wobei eine erste Verbindung (6) nur Deformationen in Richtung x, eine zweite Verbindung nur Deformationen in Richtung y und eine dritte Verbindung nur Deformationen in Richtung z zulassen.

8. Messelement nach einem der Ansprüche 1 bis 3, **dadurch gekennzeichnet, dass** die Verbindungen (6) Deformationen in mindestens zwei, vorzugsweise in drei der drei Richtungen (12) x, y und z zulassen.

9. Messelement nach Anspruch 8, **dadurch gekennzeichnet, dass** mindestens zwei, vorzugsweise drei Lichtleiter (8) für optische Abstandsmessungen zwischen den beiden Bereichen (7) der Struktur (2) angeordnet sind.

10. Messelement nach einem der vorhergehenden Ansprüche, **dadurch gekennzeichnet, dass** die Struktur (2) ein Hohlkörper mit einer zentralen Aussparung (13) ist.

11. Messelement nach einem der vorhergehenden Ansprüche, **dadurch gekennzeichnet, dass** die Struktur (2) einen Durchmesser (14) von weniger als 2.5 mm, insbesondere zwischen 1.7 und 2mm aufweist.

12. Messelement nach einem der vorhergehenden Ansprüche, **dadurch gekennzeichnet, dass** die optische Abstandsmessung mit einem interferometrischen Messprinzip, insbesondere mittels Weisslichtinterferometrie gemessen werden kann.

13. Messelement nach einem der vorhergehenden Ansprüche, **dadurch gekennzeichnet, dass** jeder Lichtleiter (8) aus einem Lichtleiterpaar besteht für photometrische Messungen.

14. Messelement nach einem der vorhergehenden Ansprüche, **dadurch gekennzeichnet, dass** die Struktur (2) mindestens in der Umgebung der Verbindungen (6) mit einer Membranhülle (15) zur Abdichtung umschlossen ist.

15. Messelement nach einem der vorhergehenden Ansprüche, **gekennzeichnet durch** einen Überlastschutz, der verhindert, dass die Enden der Lichtleiter (9) bei Überlast die gegenüberliegenden reflektierenden Flächen (10) berühren und/oder die Struktur irreversibel deformiert wird.

16. Messelement nach einem der vorhergehenden Ansprüche, **dadurch gekennzeichnet, dass** die Struktur (2) aus einer leitenden Keramik oder aus einem Halbleitermaterial, insbesondere aus Silizium besteht.

17. Verwendung des Messelements nach einem der vorhergehenden Ansprüche für invasive und/oder minimalinvasive medizinische Zwecke.

18. Verwendung des Messelements nach einem der Ansprüche 1 bis 16 oder nach Anspruch 17, **dadurch gekennzeichnet, dass** die optische Abstandsmessung mit einem interferometrischen Messprinzip, insbesondere mittels Weisslichtinterferometrie durchgeführt wird.

## Claims

1. An optical measuring element (1) for measuring forces in at least one direction (z) wherein said measuring element comprises a single-piece structure (2) with an outside wall (3) and with notches (4) introduced therein wherein said notches (4) define one or more elastically flexible zones (5) in the structure (2) being the only connection (6) between first and second regions (7) of the structure (2), and wherein for optical distance measurements between the two regions (7) of the structure (2) one or more optical fibers (8) are each attached with one end (9) thereof to a region (7) of the structure (2) such that close to and facing the ends (9) thereof reflective surfaces (10) are located which are firmly connected to another region (7), **characterized in that** the optical fibers (8) are disposed on the outside wall (3) and that each notch (4) comprises parallel edges (16).

2. A measuring element according to claim 1 **characterized in that** all notches (4) penetrate the whole structure (2) to the outside wall (3).

3. A measuring element according to any of the claims 1 or 2 **characterized in that** it comprises at least a second, preferably also a third optical fiber (8) for optical distance measurements between two distinct regions (7) of the structure (2).

4. A measuring element according to claim 3 **characterized in that** the structure (2) is subdivided by several connections (6) having elastically flexible zones (5) into at least three, preferably four distinct regions (7).

5. A measuring element according to claim 4 **characterized in that** between each two adjacent regions (7) at least one optical fiber (8) for optical distance measurements is disposed at the structure (2).

6. A measuring element according to claim 4 **characterized in that** each connection (6) between two adjacent structures (2) allows for deformations in only one of the directions (12) x, y, and z.

7. A measuring element according to claim 6 **characterized in that** the structure (2) is subdivided by three connections (6) into four regions (7) arranged in series wherein a first connection (6) allows for deformations in the x direction only, a second connection allows for deformations in the y direction only, and a third connection allows for deformations in the z direction only.

8. A measuring element according to any of the claims 1 to 3 **characterized in that** the connections (6) allow for deformations in at least two, preferably in three of the directions (12) x, y, and z.

9. A measuring element according to claim 8 **characterized in that** for optical distance measurements at least two, preferably three optical fibers (8) are arranged between the two regions (7) of the structure (2).

10. A measuring element according to any of the preceding claims **characterized in that** the structure (2) is a hollow body having an central cavity (13).

11. A measuring element according to any of the preceding claims **characterized in that** the structure (2) has a diameter (14) of less than 2.5 mm, in particular between 1.7 and 2 mm.

12. A measuring element according to any of the preceding claims **characterized in that** the optical distance measurement can be performed by an interferometric measurement principle, in particular by means of white light interferometry.

13. A measuring element according to any of the preceding claims **characterized in that** each optical fiber (8) consists of a pair of optical fibers for photometric measurements.

14. A measuring element according to any of the preceding claims **characterized in that** for the purpose of sealing the structure (2) is surrounded by a membrane sheath (15) at least in the region of the connections (6).

15. A measuring element according to any of the preceding claims **characterized in** having an overload protection that prevents the ends of the optical fibers (9) from contacting the opposite reflective faces (10) in case of an overload and/or prevents an irreversible deformation of the structure.

16. A measuring element according to any of the preceding claims **characterized in that** the structure (2) is made of a conductive ceramic or of a semiconductor material, in particular of silicon.

17. The use of a measuring element according to any of the preceding claims for invasive and/or minimally invasive medical purposes.

18. The use of the measuring element according to any of the claims 1 to 16 or claim 17 **characterized in that** the optical distance measurement is carried out using an interferometric measurement principle, in particular by means of white light interferometry.

## Revendications

1. Un élément de mesure optique (1) pour la mesure des forces dans au moins une direction (z), élément de mesure comprenant une structure d'une seule pièce (2) avec une paroi extérieure (3) et avec des encoches (4) qu'on y a introduites dans lequel lesdites encoches (4) définissent dans la structure (2), une ou plusieurs zones (5) aptes à un fléchissement élastique et qui sont la seule liaison (6) entre une première et une deuxième région (7) de la structure (2), et dans lequel pour la mesure de distance par voie optique entre lesdites deux régions (7) de la structure (2) une ou plusieurs fibres optiques (8) sont fixées chacune à l'une de ses extrémités (9) à une région (7) de la structure (2) d'une telle manière que près de et en face de ses extrémités (9) sont disposées des surfaces réfléchissantes (10) fermement reliées à une autre région (7), **caractérisé en ce que** lesdites fibres optiques (8) sont arrangées à ladite paroi extérieure (3) et **en ce que** chacune des encoches (4) comprend des bords parallèles (16).

2. Un élément de mesure selon la revendication 1 **caractérisé en ce que** toutes les encoches (4) pénètrent toute la structure (2) jusqu'à la paroi extérieure (3).

3. Un élément de mesure selon l'une quelconque des revendications 1 ou 2 **caractérisé en ce qu'**il comprend au moins une deuxième, préférentiellement aussi une troisième fibre optique (8) pour la mesure de distance par voie optique entre deux régions (7) distinctes de la structure (2).

4. Un élément de mesure selon la revendication 3 **caractérisé en ce que** la structure (2) est subdivisée en au moins trois, de préférence quatre régions (7) distinctes par plusieurs liaisons (6) ayant des zones (5) aptes à un fléchissement élastique.

5. Un élément de mesure selon la revendication 4 **caractérisé en ce qu'**entre chaque deux régions (7) adjacentes est disposée à la structure (2) au moins une fibre optique (8) pour la mesure de distance par voie optique.

6. Un élément de mesure selon la revendication 4 **caractérisé en ce que** chaque liaison (6) entre deux structures (2) adjacentes ne permet que des déformations dans une seule des directions (12) x, y, et z.

7. Un élément de mesure selon la revendication 6 **caractérisé en ce que** la structure (2) est subdivisée par trois liaisons (6) en quatre régions (7) disposées en série dans lequel une première liaison (6) ne permet que des déformations dans la direction x, une deuxième liaison ne permet que des déformations dans la direction y et une troisième liaison ne permet que des déformations dans la direction z.

8. Un élément de mesure selon l'une quelconque des revendications 1 à 3 **caractérisé en ce que** les liaisons (6) permettent des déformations dans au moins deux, de préférence trois des directions (12) x, y, et z.

9. Un élément de mesure selon la revendication 8 **caractérisé en ce que** pour la mesure de distance par voie optique au moins deux, de préférence trois fibres optiques (8) sont arrangées entre les deux régions (7) de la structure (2).

10. Un élément de mesure selon l'une quelconque des revendications précédentes **caractérisé en ce que** la structure (2) est un corps creux ayant une cavité centrale (13).

11. Un élément de mesure selon l'une quelconque des revendications précédentes **caractérisé en ce que** la structure (2) a un diamètre (14) inférieur à 2,5 mm, particulièrement entre 1,7 et 2 mm.

12. Un élément de mesure selon l'une quelconque des revendications précédentes **caractérisé en ce que** la mesure de distance par voie optique peut être effectuée selon un principe de mesure interférométrique, en particulier au moyen de la interférométrie à lumière blanche.

13. Un élément de mesure selon l'une quelconque des revendications précédentes **caractérisé en ce que** chaque fibre optique (8) consiste en une paire de fibres optiques pour des mesures photométriques.

14. Un élément de mesure selon l'une quelconque des revendications précédentes **caractérisé en ce que** la structure (2) est entourée par une gaine membraneuse (15) au moins dans la région des liaisons (6) afin de l'étancher.

15. Un élément de mesure selon l'une quelconque des revendications précédentes **caractérisé en ce qu'**il possède une protection de surcharge empêchant que les extrémités des fibres optiques (9) touchent les faces réfléchissantes (10) disposées en face d'eux en cas de surcharge, et/ou empêchant une déformation irréversible de la structure.

16. Un élément de mesure selon l'une quelconque des revendications précédentes **caractérisé en ce que** la structure (2) est fabriquée d'une céramique conductrice ou d'un matériau semi-conducteur, particulièrement de silicium.

17. L'utilisation d'un élément de mesure selon l'une quelconque des revendications précédentes à des fins médicales effractives et/ou mini-invasives.

18. L'utilisation de l'élément de mesure selon l'une quelconque des revendications 1 à 16 ou la revendication 17 **caractérisé en ce que** la mesure de distance par voie optique est effectuée en utilisant un principe de mesure interférométrique, en particulier au moyen de la interférométrie à lumière blanche.
